Europäisches Patentamt

European Patent Office

Office européen des brevets

(11) Publication number: **0 324 711**
**A2**

## (12) EUROPEAN PATENT APPLICATION

(21) Application number: **89710002.0**

(22) Date of filing: **10.01.89**

(51) Int. Cl.⁴: **A 61 B 17/22**

(30) Priority: **11.01.88 US 142124**
**27.12.88 EP 88121698**

(43) Date of publication of application:
**19.07.89 Bulletin 89/29**

(84) Designated Contracting States:
**CH DE ES FR GB IT LI**

(71) Applicant: **DORNIER MEDIZINTECHNIK GMBH**
**Postfach 1128**
**D-8034 Germering 1 (DE)**

(72) Inventor: **Chvapil, Milos, Dr.**
**The University of Arizona Health Science Center**
**Tucson Arizona 85724 (US)**

**Haupt, Gerald**
**The University of Arizona Health Science Center**
**Tucson Arizona 85724 (US)**

(74) Representative: **Landsmann, Ralf, Dipl.-Ing.**
**DORNIER GMBH - Patentabteilung - Kleeweg 3**
**D-7990 Friedrichshafen 1 (DE)**

(54) Apparatus for enhancing wound and fracture healing.

(57) An apparatus for enhancing wound or fracture healing comprising a source (32) for acoustic shock waves and a device for coupling them into a site where healing is desired.

Fig. 2

Bundesdruckerei Berlin

Description

## APPARATUS FOR ENHANCING WOUND AND FRACTURE HEALING

### BACKGROUND OF THE INVENTION

The present invention relates to the healing of bone fractures, non-unions, and pseudoarthroses, and to the regulation of wound healing.

Bones are important organs of the human body. On the one hand, they act as supports, and on the other hand, they serve as a storage facility for calcium and phosphorus. These two elements are present in bone in the form of apatite, a mineral. Depending upon other conditions in the body, calcium and phosphorus may be introduced or removed by hormonal regulation. For example, the body keeps a relatively constant level of calcium in the blood, because important biological activities such as contraction of muscles, beating of the heart, and clotting of blood require quite constant blood levels of calcium.

When the blood calcium level drops, more calcium is taken out of the bones to maintain the appropriate level. When the blood calcium returns to normal, increased amounts of calcium are no longer taken from the bones.

Living bone contains a protein framework made of collagen (the osteoid matrix) in which the calcium salts are deposited. Bone, like many other tissues of the body, is constantly rebuilt or remodeled. Components of old bone are torn down, resorbed and replaced with new bone. The cells affecting the removal of calcium, phosphorus and collagen from bones are the osteoclasts. Those which deposit collagen, calcium, and phosphorus in bone are osteoblasts. Thus, the building of bones by the osteoblasts takes place by the initial formation of the osteoid matrix which consists primarily of collagen (a fibrous protein) which is surrounded by proteoglycans and glycoproteins. Subsequent to formation, the matrix is enclosed by apatite to combine the desirable properties of the elastic fibers and the hard mineral substance.

As mentioned above, the osteoclasts are bone decomposing cells. They are generally multinuclear cells provided with numerous different enzyme activities. The enzymes and the acidity of the osteoclasts facilitate the decomposition of bone by on the one hand, dissolving the mineral thereby releasing ionic calcium and phosphate, and on the other, decomposing the organic matrix.

In intact, healthy bones, osteoblasts and osteoclasts coexist and both are active. This means that in healthy bone, a dynamic equilibrium is present between the osteoclast activity (bone decomposition) and osteoblast activity (bone building). In a healthy young person these processes are balanced, thus implying that the average bone mass is the result of an equilibrium which depends on the age and physiological condition of the person under consideration. Interference with this equilibrium leads either to a decrease or increase in bone mass.

When a bone is fractured, there is generally tearing of small blood vessels causing bleeding at the fracture site. Bleeding occurs between the bone fragments and into the marrow cavity, as well as under the periosteum (the thin, tough fibrous membrane which covers the outer surface of the bone). This collection of blood and serum forms a clot which is called the "fracture-hematoma". The fracture-hematoma plays a very important role in fracture repair. The fracture-hematoma first undergoes a repair process which is similar to that which occurs in injuries of other tissues. This is known as organization of the clot. In the first few days there is rapid growth into the clot of cellular granulation tissue composed of fibroblasts and new fine capillary blood vessels. In about a week, small areas of young bone begin to be formed in an irregular pattern about these capillary blood vessels.

In addition to this organization of the clot by fibrous granulation tissue, there is a proliferation or multiplication of both osteoblasts and chondroblasts (cartilage-forming cells). Both new bone and new cartilage are laid down. This eventually forms the "callus", not only between the bone fragments but also under the periosteum and around the fracture site. At the same time that this new cartilage and new bone is being formed, all devitalized bone from the ends of the fracture fragments is being absorbed by osteoclasts, to be replaced by new bone.

The new cartilage which is formed in the callus is usually ultimately transformed into bone. Sometimes, however, cartilage formation predominates and little or no new bone is formed. This can happen when there is too much mobility between the fracture fragments. As a result, the fracture does not unite and a false joint (pseudoarthrosis) results. For this reason, immobilization of the fragments to prevent motion between them is essential during the healing stage of a fracture.

The primary callus which is first formed is fibrous at first and later becomes ossified. Primary bony callus is usually complete in some five to seven weeks after injury, but mineralization by deposit of calcium salts is not completed. It usually takes about two to four weeks longer, on the average, to complete mineralization so that the fracture fragments are united by solid bone. This time schedule varies for the different bones and can be much longer before bony union occurs. While this is referred to as "clinical union", this primary bone callus is a primitive type of solid bone and is actually only a temporary repair. Eventually, the adult type of lamellar bone must be formed in its place, and the original microscopic architecture of the bone must be restored.

Adult lamellar bone with its Haversian systems and medullary cavity forms very slowly. It requires for its formation the existing model of primitive new bone which the primary bony callus supplies. By the slow and continuous process of absorption and replacement, new adult bone is laid down in place of primary callus.

Eventually, the parallel lamellar pattern of adult bone with its Haversian systems is restored and the marrow cavity which was temporarily occluded is recanalized. Excess callus is absorbed, thus restoring the original contour of the bone. It is only at this stage that anatomical healing is complete. This entire process takes a year or even longer for a major bone.

Unfortunately, various circumstances may interfere with fracture repair. One of the common interferences is loss of the fracture-hematoma. Since the fracture-hematoma plays the stellar role in the healing of a fracture, if this hematoma does not form, or is lost or resorbed, the normal healing process is delayed or sometimes does not even occur. For example, this happens in compound fractures in which bleeding is to the outside and not within a closed space and thus no clot forms, and in surgical reduction of a fracture, for the same reason. This is one reason why closed reduction of a fracture is preferred if it is possible by this means to obtain and maintain good alignment and contact of the fracture fragments.

The skin is another important organ of the human body. The skin consists of an outer layer or epidermis, and an underlying layer referred to as the dermis. The skin acts as mechanical protection, a means of conserving water, a generalized sensory organ, and is important in temperature regulation. Until recently, the skin had been viewed as largely only a barrier between the interior of the body and the outside world. However, very recent research has suggested that the skin may have other functions, including an active role in the body's immune system.

Healthy, intact skin is an essential element in the prevention of infection. The longer a wound is open, and the deeper the wound, the more likely it is that infection may occur before healing is complete.

Nevertheless, it is important that the covering on a wound, by either epithelial cells (in shallow wounds) or by scar (in deep wounds), be uniform and have an appearance that is as close as possible to surrounding areas. While there are no abnormalities with healing of shallow wounds, excessive formation of scar tissue in deep wounds is reflected in hypertrophic scars, keloids, and wound contractures, resulting in deformities.

In addition to noncomplicated skin wounds, under certain conditions healing is delayed due to diabetes, exposure of the skin to radiation, or immunosuppression. Examples of delayed healing are: leg ulcers and decubitus ulcers (an ulceration caused by prolonged pressure in a patient allowed to lie too still in bed for a long period of time).

Because of the relatively long duration of impaired function resulting from a bone fracture, and the tendency for infection and other complications which may result from a skin wound, various methods employing physical influences have been suggested in the art for accelerating healing. Most of the physical factors, such as electrical stimulation, electromagnetic fields, piezoelectricity, ultrasound or mechanical influences such as intermittent tension, effects of immobilization and continuous passive motion and micromovement were tested in biological systems involving bone growth, fracture healing, or skin wound healing. Although the actual mechanism or mechanisms involved in the stimulation of the wound repair by the above listed factors are not understood, several hypotheses were suggested. Cell proliferation, the activation of cells with enhanced deposition of structural macromolecules by these cells, and also the faster remodeling of the treated tissue may explain these effects. These hypotheses include the effect of the transduced physical energy on the structural and functional changes of molecules at the plasma membrane level (cAMP, cGMP, ATPase, etc.), which by a second messenger mechanism promote cell mitosis and enhance cell activity to synthesize structural macromolecules. It may well be an induced effect of physical forces on the mediators or activators of cell activity, such as release of serotonin or histamine by mast cells.

An example of such a physical influence is Duarte, U.S. Patent No. 4,530,360, which describes an apparatus and method for healing bone fractures, pseudoarthroses and the like with the use of ultrasound. An ultrasound transducer, in contact with the skin of the patient, transmits ultrasound pulses to the site of the bone defect. The nominal frequency of the ultrasound is 1.5 MHz, the width of each pulse varies between 10 and 2,000 microseconds, and the pulse repetition rate varies between 100 and 1000 Hz. The power level of the ultrasound is maintained below 100 milliwatts per square centimeter. It is stated therein that treatments which last about 20 minutes per day have been found to heal certain defects in less than two months.

Another method referred to in Duarte which has been employed in an attempt to accelerate healing is the application of direct current, on the order of 20 microamperes, at the site of a fracture. The cathode is usually applied at the site of the defect, whereas the anode is placed somewhere in the adjacent tissue or on the skin of the patient. Unfortunately, such arrangements are totally or partially invasive, which raises the concomitant possibility of infection. With non-invasive techniques, such as causing an externally generated electromagnetic field to pass through the fracture site to induce a current, precise alignment of the coils relative to the area to be treated is required, as well as treatment being required for 12 to 16 hours a day.

In the past, acoustic shock waves have been used to treat patients, but not in relation to bone or skin. For example, shock wave treatments have been widely used to break up kidney stones to avoid invasion of the patient's body by instruments. Such treatments are described in publications such as United States Patent No. 3,942,531 to Hoff et al. and Extracorporeal Shock Wave Lithotripsy, 1982, edited by Christian Chaussy and published by Karger AG of Basel, Switzerland. Generally, shock waves are generated exteriorly of the patient's body in a medium such as water and transmitted into the patient's body with suitable coupling to minimize energy absorption at the interface with the patient's skin. As pointed out in Chaussy, the shock waves (which differ from ultrasound wave inputs in that they have a very steep compression pressure rise front and little or no tension component) may travel through normal soft body tissue (except the lungs) at high pressure amplitudes without materially injuring the tissue.

3

SUMMARY OF THE INVENTION

The present invention is an apparatus for enhancing wound or fracture healing. The apparatus comprises directing energy in the form of acoustic shock waves to a site where healing is desired, the amount of energy applied being sufficient to accelerate healing. The invention comprises the industrial application (like manufacture, production, import, sale) of a known Lithotripter - not the nonprofit application like the use by a physician.

BRIEF DESCRIPTION OF THE DRAWINGS

FIG. 1 provides a graphical comparison of features of a shock wave and an ultrasound wave.

FIG. 2 schematically illustrates an apparatus set-up for generating shock waves outside a patient's body and using them to stimulate fracture or wound healing.

FIG. 3 is a graph comparing the influence of shock waves on the healing of split thickness wounds in the pig model.

FIG. 4 is a graph comparing the effect of shock waves on the contraction of deep wounds in piglets.

FIG. 5 is a graph comparing the effect of shock waves on granulation tissue formation of deep wounds in piglets.

FIG. 6 is a graph comparing the influence of shock waves on the healing of fractured humeri in rats.

**DETAILED DESCRIPTION OF PREFRRED EMBODIMENTS**

By the present invention, an apparatus for enhancing wound or fracture healing is provided. Relatively low level shock waves stimulate the cells of the wound or fracture and consequently enhance healing and stimulate healing of a wound. However, it must be emphasized that the healing is dose-dependent, and if the dosage is too large, the treatment will be counterproductive by resulting in inhibition of cells due to cell destruction and necrosis.

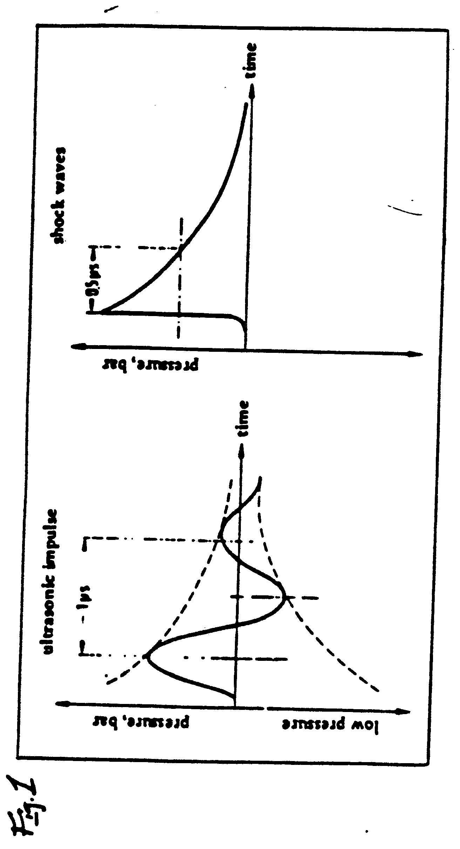

The nature of the shock waves utilized in the present invention will be explained with reference to Fig. 1 which depicts an idealized format characteristics of both an ultrasonic wave impulse which is not suitable for the practice of the present invention and an acoustic shock wave which is suitable. In each instance, the wave form is displayed in a pressure versus time diagram where pressure is indicated by the vertical direction and time is indicated by the horizontal direction. Pressures above ambient are located above the "time" axis and pressures below ambient are located below the "time" axis.

The ultrasound wave at the left of Fig. 1 is sinusoidal in nature in that the pressure rises regularly to a maximum value above ambient, falls regularly through ambient and on to a minimum value below ambient, and then rises regularly again. It will be understood that the portion of the curve above the "time" axis represents "compression" (shrinking) conditions in the medium in which the wave moves and the portion of the curve below the "time" axis represents "tension" (expanding) conditions in the medium.

The idealized shock wave at the right of Fig. 1 has a single pressure spike with very steep onset and a more gradual relaxation. Moreover, it has no "tension" component in that pressures throughout are above ambient.

Some parameters associated with the shock waves which are emphasized in practicing the invention will promote a more complete understanding. With regard to the steep compression wave front, a typical rise time (period from onset to peak) is less than about $10^{-7}$ seconds. The maximum pressure attained will be relatively low, appropriate values being in the range of about 20 bar to about 800 bar with values less than 800 bar and preferably less than 600 bar being favored. The overall duration of the whole wave event can be expected to be on the order of six microseconds or less, and the wave will have little or no "tensile" (pressure below ambient) component. In this latter regard, any "tensile" component should be less than 25%, and preferably less than 10%, of the maximum pressure amplitude, and any oscillation should be minimized.

The absence of extensive tension wave components in shock waves is important from the standpoint of undesired tissue damage in patients. In the case of enhancement of fracture healing it is important that the waves pass through the intervening soft tissue without substantial trauma. Similarly, it is important that wound tissue not be disrupted during attempts to enhance the healing process. Tension wave conditions are tolerated less well than compression wave conditions, and pressures far below ambient tend to cause damage to tissue. Hence, these components should be minimized both in amplitude and extent where possible.

The frequency spectra corresponding to the two types of waves diagrammed in Fig. 1 also are significant with respect to the biological effects the waves produce. A shock wave may be considered as a complicated composition of longitudinal waves covering a very large frequency range. In contrast therapeutic ultrasound represents a more or less short wave train of ultrasonic waves. Due to the extremely short rise time, the shock wave is a unique means to realize high compression zones within the tissue without producing heat and cellular degradation. Ultrasonic waves of a comparable energy regime may produce heat and cellular degradation, whereas shock waves have far less effect on the patient's body.

To further illustrate the differences between the use of shock waves in the present invention versus the use of pulsed ultrasound as recommended in Duarte, the following Table 1 is provided which lists various pertinent parameters:

TABLE I

| | Pulsed Ultrasound | Shock Waves |
|---|---|---|
| intensity | 0.1 W/cm$^2$ | 200 kW/cm$^2$ |
| maximum pressure amplitude | 0.5 bar | 800 bar |
| frequency | 1.5 MHz | 0.5 MHz (average frequency) |
| rise time | 170 ns | < 50 ns |
| pulse length | 10-2000 $\mu$ s | - 6 $\mu$ s |
| repetition rate | 10$^2$ - 10$^3$ Hz | - 1 Hz |
| wavelength (H$_2$O) | 1 mm | - 3 mm (average wavelength) |
| energy density (H$_2$O) | 0.7x10$^{-6}$ WS/cm$^3$ | 1.2 WS/cm$^3$ |
| displacement amplitude | 0.02 $\mu$m | 100 $\mu$ m |

Several important points should be appreciated from the above. Initially, the differences in energy density and maximum pressure amplitude should be noted. Energy is equal to power multiplied by time. Duarte provides a pulse length (i.e., time) of approximately the same or longer duration as the present invention, and a pulse repetition rate which is 10$^2$ to 10$^3$ times larger than in the case of shock waves. However, due to the great difference in the intensities (see Table I) the time average intensity of the shock waves is 10$^3$ times larger than the time average intensity of the pulsed ultrasound. Also the very significant difference in the displacement amplitudes (Table I, bottom row) should be noted.

Moreover, any attempt to modify Duarte to provide comparable energy using ultrasound would be less successful physiologically. Essentially, shock waves succeed by supplying high power in a short time, due to a rapid rise time to the maximum pressure amplitude. To obtain the same amount of energy from ultrasound, it would be necessary to supply relatively low power for a longer time. The result of the longer time of application would be storage of energy in the tissue, with concomitant heating and tissue degradation.

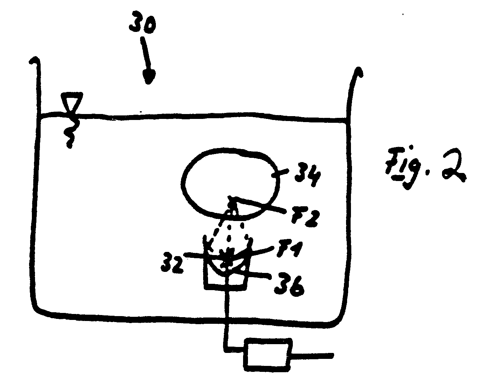

Fig. 2 shows a diagrammatic view of an apparatus setup for enhancing fracture or wound healing by shock wave treatments. A shock wave source 32 and a patient 34 are positioned in a container 30 of water which preferably is degassed. This source 32 is to be understood as a spark gap device driven by an electrical condenser which releases its energy in a very short time. An arc arises between the electrodes which vaporizes the water surrounding the arc's path, establishing a plasma-like state. The result is an explosion-like vaporization of the water which produces a shock wave that spreads out in a circular fashion.

With this embodiment, the shock wave source is arranged in the focus F1 of an ellipsoid of revolution 36. Shock waves emanating from the shock wave source are concentrated in the focus F2 where the fracture or wound to be treated is located. Since the impedance in the water bath is about the same as the speed of sound in body tissue, the illustrated arrangement advantageously couples the shock waves into the patient's body with little reflection of energy at the body-water interface and with little likelihood of injury to the patient, all in a manner well known to those skilled in medical applications of shock waves.

The illustrated arrangement also permits safe relative movement between the shock wave source and parts of the patient's body, so that the shock waves may be focused to the wound or fracture where the enhancement of wound or fracture healing is desired.

Other physical arrangements are of course possible. For example, instead of using a water bath for the patient, it has been proposed to interpose a suitable diaphragm or membrane between the patient's skin and a water cushion in which shock waves are generated. With such arrangements, a flexible bellows can be used for coupling so that relative movements can be accomplished for focusing the shock waves as required.

Other systems for generating shock waves may of course be used if desired. It is known, for instance, that laser beam energy focused at the focal point F1 can produce an acoustical shock wave emanating from that point.

Other energy inputs (e. g., electromechanical sources and piezoelectric generators) and other focusing techniques (e.g., hemispheres, lenticular focusing by a lens or lens system) also are possible. An array of piezoelectric devices arranged on a spherical segment or concave support, and suitably driven to provide shock wave outputs, can provide shock waves which converge or focus at a point or zone spaced away from the support; and such an arrangement has been proposed for introducing shock waves into a patient's body.

It is important to distinguish between the induction of bone growth, i.e., causing bone growth to begin, versus stimulation, i.e., enhancing a process which is already occurring. The present invention will be of lesser value in the case of osteoporosis, where no bone growth whatsoever would occur unless such is induced by

5

the application of acoustic shock waves. Moreover, in smooth fractures such as stress fractures, the low resulting pertubation results in minimal hematoma formation and essentially no formation of bone. In these situations, higher energy levels than those employed in the present invention may be necessary to induce bone growth. By the application of higher energy acoustic shock waves, new bone growth can be induced, thereby resulting in healing which would otherwise be delayed or would not occur.

The number of acoustic shock waves and whether a series of treatments should be administered will depend upon the age of the individual and the particular bone or wound involved. For example, an older individual may require a longer duration of treatment to stimulate the cells to an acceptable rate of healing. Considering the HM 3 shock wave generator system (Dornier Medizintechnik GmbH, Germering, Federal Republic of Germany), for example, a dose of about 14 to 18 kV (corresponding to peak amplitude pressures of less than about 600 bar (60 MPa)) times fewer than 500 acoustic shock waves in multiple applications over a period of time will generally be effective for a bone such as a femur or iliac. To avoid bleeding and damage of tissue, the maximum should not exceed 18 kV (corresponding to a focal peak amplitude pressure of approximately 600 bar (60 MPa)). Generally, a maximum of about 500 acoustic shock waves would be applied in each application. In general, treatments will be applied approximately every 2-5 days until the healing process is significantly stimulated. The object of the treatment is to bring the cells out of the "inertia" which is part of the delayed healing phenomena.

In order to further illustrate the present invention and the advantages thereof, the following specific examples are given, it being understood that these examples are intended only to be illustrative without serving as a limitation on the scope of the present invention.


## EXAMPLE 1


Wound Healing with Acoustic Shock Waves

In order to demonstrate the present invention and the importance of the level of energy input of the shock waves on wound healing, the following study was conducted on the healing of split thickness wounds in a standardized model in piglets. The model is described in detail in Chvapil et al, J. Surg. Res., 44, 266-276 (1988). This model quantitates the degree of reepithelialization of the wound by morphometric evaluation of epithelial coverage of the wound area.

Three Yorkshire pigs with a body weight of 9-11 kg were used. Split thickness wounds (1 x 1 cm area, 0.3 - 0.5 mm deep) were inflicted with an electrokeratome. Nine to 22 wounds were inflicted in each pig totaling 43 wounds in the entire study. The wounds were covered with waterproof Op-Site adhesive film. Using the Dornier experimental device XL1 the shock waves were applied at 14 or 18 kV generator voltage (a peak amplitude pressure of about 500 and 800 bar, and an intensity of about 75 and 192 kW/cm$^2$, respectively) and 10 to 1000 shock waves (SW) per treatment. The actual focus pressure in the XL1 is higher than in the lithotripters of the HM series, which are used for ESWL and have a different ellipsoid. There were two to four wounds used per one treatment modality in each pig, as is evident from Table 1. Before the actual exposure of pigs to shock waves, the animals were anesthetized with halothane for easy handling and placed in the 35° ± 0.5°C water bath into a restrainer. Two to four wounds per pig remained untreated as control wounds. To achieve equal shock wave application to the complete wound area, the wounds were treated in the blast path 0.7 cm above the focus point F2. This was established by treating normal skin with 1000 SW to produce a reddened area having the same size as the wounds, so as to standardize the technique of delivering shock waves to a treated skin area.

After the treatment, the Op-Site occluded wounds were rinsed with a penicilline-streptomycine solution to avoid infection due to possible leakage of water onto the wound. The animals were sacrificed after 54 hours and the wounds were fixed in formalin. Four random cuts of each wound were stained with hematoxylin-eosin. The magnitude of epithelialization of the wound was counted in four sections in relation to the whole length of the wound. The percentage of epithelialized wound area in individual treatment groups were statistically evaluated with Duncan's multiple range test (Duncan, D.B.: Multiple Range and Multiple F-Tests, Biometrics, 11:1, 1955).

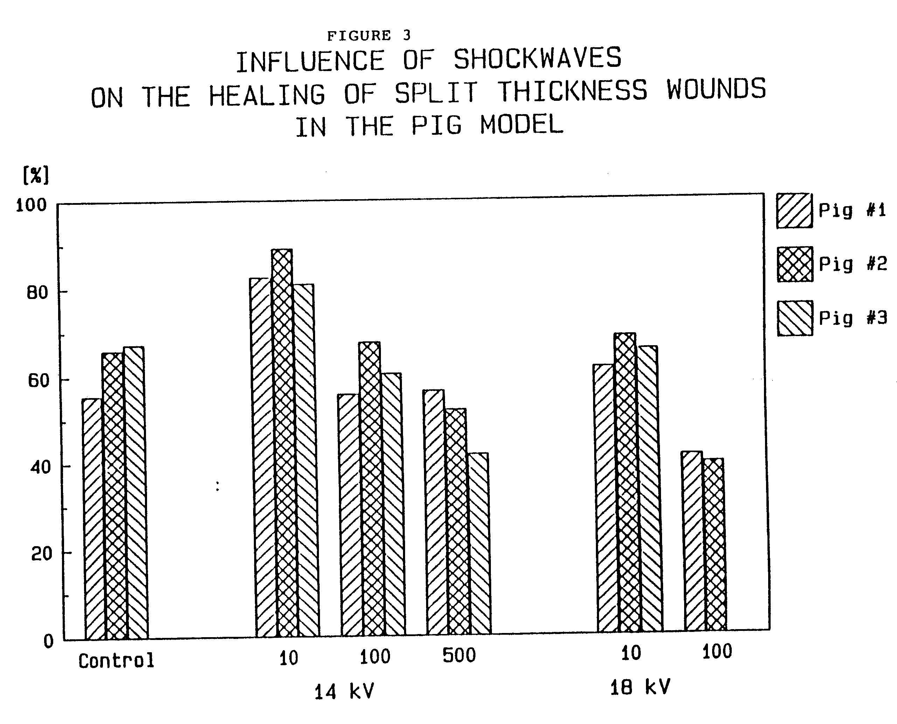

After 54 hours the normal rate of reepithelialization in split thickness wounds varies from 50 to 70% depending on the age, weight and nourishment of the animal and the density of hair follicles. In this example the control wounds inflicted in three piglets showed 56 to 67% of reepithelialization. Wounds treated with 100 SW at 500 bar pressure and 75 kW/cm$^2$ intensity and 10 SW at 800 bar pressure and 192 kW/cm$^2$ intensity ended up with similar rates of epithelialization. With increased numbers of SW (500 SW and 1000 SW at 14 kV, 100 SW at 18 kV) healing was significantly inhibited ($p < 0.05$), while with low dose treatment (10 SW at l4 kV) reepithelialization was significantly enhanced ($p < 0.05$) in two of three independent experiments. Thus, the reaction of the epithelial cells of the skin wound followed a dose-response curve, as documented in Figure 3. The variability of the results is shown in Table 1, which also indicates the number of individual measurements per each treatment.

The wounds exposed to various shock wave treatments, as well as the control wounds, were evaluated by standard histological methods for the overall morphological changes. The control wounds and the wounds treated with high doses had fibrin deposits, multiple dense infiltrations of polymorphonuclear leukocytes and necrotic debris on the epidermis. Moderate signs of acute and chronic inflammation were observed in the dermis. Vessels with internal swelling and prominent endothelial cells were noted as well as many active fibroblasts and perivascular inflammatory infiltrates.

TABLE 1

EFFECT OF SHOCK WAVES AT FIVE ENGERGIES ON THE EPITHELIALIZATION RATE OF PARTIAL THICKNESS WOUNDS IN PIGLETS

| Volts - number of pulses | Group | | | | | | | |
|---|---|---|---|---|---|---|---|---|
| | | Pig 1 | | | Pig 2 | | | Pig 3 |
| | $N^{2)}$ | | Epithelialization[1] | $N^{2)}$ | | Epithelialization | $N^{2)}$ | Epithelialization |
| Control | | 14 | $55.7 \pm 10.1^{a4)}$ | 6 | | $66.0 \pm 1.79^{a}$ | 7 | $67.3 \pm 9.5^{a}$ |
| 14 kV - 10 | | 11 | $82.5 \pm 11.7^{b}$ | 7 | | $89.1 \pm 15.2^{b}$ | 6 | $81.0 \pm 21.3^{a}$ |
| 14 kV to 100 | | 13 | $55.9 \pm 9.8^{a}$ | 7 | | $67.6 \pm 15.5^{a}$ | 8 | $60.5 \pm 18.6^{b}$ |
| 14 kV 500 to 1000[3] | | 7 | $56.6 \pm 10.5^{a}$ | 5 | | $52.2 \pm 7.9^{a}$ | 4 | $41.8 \pm 3.9^{c}$ |
| 18 kV - 10 | | 16 | $61.8 \pm 16.7^{a}$ | 7 | | $48.0 \pm 27.3^{a}$ | 4 | $65.8 \pm 10.4^{a,b}$ |
| 18 kV - 100 | | 15 | $41.4 \pm 10.0^{c}$ | 6 | | $39.7 \pm 16.6^{c}$ | | |

Variability is given by $X \pm SD$
2 Number of evaluated wound sections
3 500 pulses in pig #1 and #3, 1000 pulses in pig #2
4 Letters refer to statistical significant difference at 95% confidence limit calculated by Duncan's Multiple Range test. Number with different letters are significantly different.

EP 0 324 711 A2

In low dose treated wounds less fibrin, less necrotic debris and less polymorphonuclear leukocytes were found. The upper dermis showed increased numbers of small capillaries with increased numbers of macrophages in perivascular spaces. Also, the newly formed epithelial layer was four to five cells thick, almost twice as thick as in control wounds or medium energy shock wave treated wounds. Thus, the wounds treated with low doses not only showed a higher rate of reepithelialization, but also less fibrin, necrotic debris and inflammatory cells on the surface.

This example demonstrates that the application of shock waves can influence the healing of wounds in a dose-dependent fashion. While higher energies of shock waves (in number or generating voltage) result in inhibition, reepithelialization is stimulated with low energy treatment. Intermediate energies ranging from 100 SW at 14 kV and 10 SW at 18 kV were ineffective. Thus, shock waves, administered at certain levels of energies promote the repair process, cell kinetics or other phenomena involved in the wound healing.

## EXAMPLE 2

In order to further demonstrate the ability of shock waves to promote healing of wounds, the following study was conducted on wounds in which delayed healing was caused by irradiation.

Two Yorkshire piglets of 16 kg were used. The piglets were anesthetized via halothane, and intubated. The dorsal surface was shaved and prepped with 70% aqueous ethanol. Both piglets were irradiated by electron beam on two areas, each 7 cm x 10 cm, on the dorsal surface, left side and right side, below the ribs. Irradiation in each area was with 1500 rads. The areas on piglet 1 were designated areas 1 and 2, and on piglet 2, areas 3 and 4.

Two days after irradiation, an electrokeratome was used to administer shallow wounds. Four shallow wounds, 2 cm x 2 cm and 400 microns deep, were administered in each irradiated area. Wounds on piglet 1 were covered with Tegaderm and a stockinette dressing was placed on the piglet. While still anesthetized, each wound in area 3 on piglet 2 received 200 shock waves at 14 kV using the Dornier XL1 at time 0. Piglet 2 was then dressed the same as piglet 1.

No lithotripsy was administered to the wounds in area 1. The wounds in areas 2 and 4 received the above lithotripsy treatment 36 hours after wounding. The wounds in area 3 received another lithotripsy treatment as above 24 hours after wounding.

Fifty four hours after wounding, all wounds were removed to create two wounds per area approximately 2 cm x 6 cm in size. Time 0 volume measurements and wound contraction tracings were obtained for all deep wounds. Lithotripsy treatments and dressings were as above. Volume measurements and wound contraction tracings were taken every four days until the piglets were sacrificed twenty days after infliction of the deep wounds.

The exposure of split thickness wounds to low energy shock waves resulted in a statistically significant enhancement of reepithelialization in pre-irradiated skin (Table 2). The magnitude of the healing was increased 50%, based on the analysis of 16 sections made of two wounds exposed to lithotripsy and compared to the same number of analyzed sections and wounds not exposed to shock waves.

TABLE 2

| Treatment | % Skin Wound Re-epithelized (X + SD) | | Number of measurements |
|---|---|---|---|
| Control, untreated | 53.0 ± 14.6 | p < 0.05 | 16 |
| Lithotripsy at 36 hours 14 kV, 200 shocks | 77.2 ± 18.7 | | 16 |

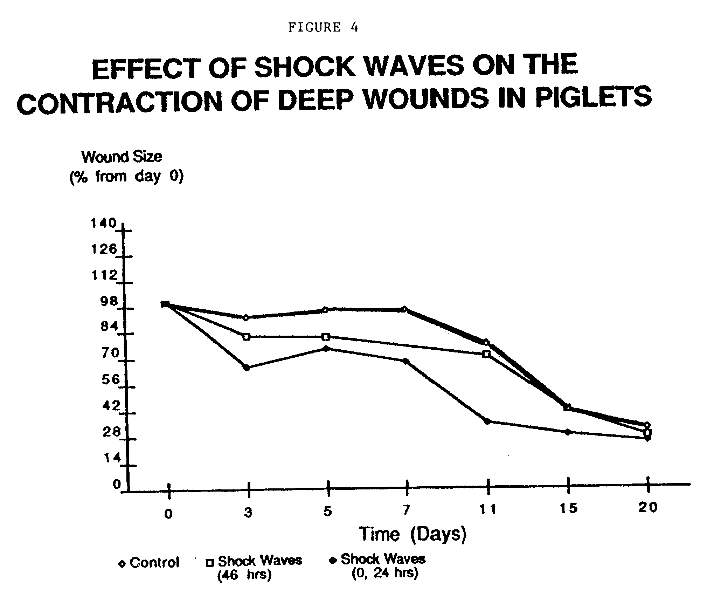

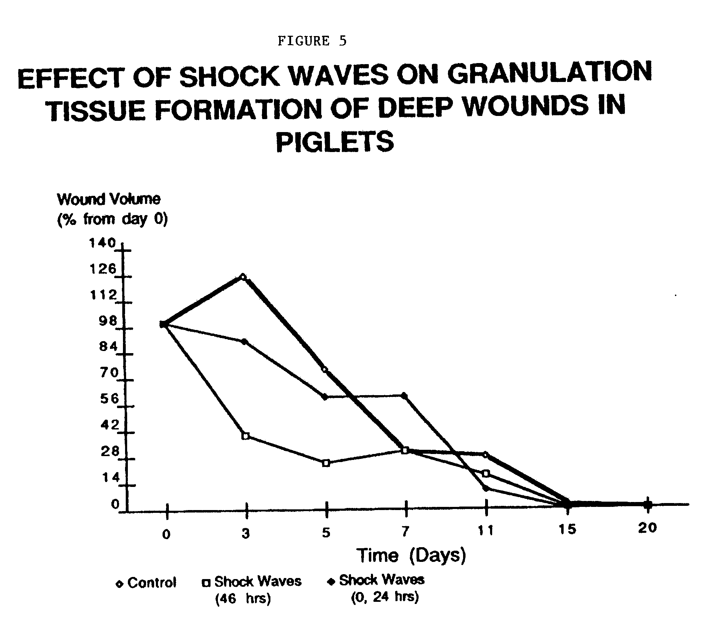

The effect of shock waves on the healing of deep wounds was determined by studying the dynamics of the wound contraction and the filling of the wound with granulation repair tissue. The data are shown in Figures 4 and 5. Each point is the average of two wound measurements. The graphs show that the healing dynamics, as reflected by the two parameters, have been enhanced by the exposure of the wounds to the shock waves. The acceleration of the wound contraction or formation of the repair tissue by shock waves is more evident at the early stages of the healing process (up to 7-10 days), while at 20 days the control wounds and lithotripsy treated wounds reach the same healing stage.

From these results it can be concluded that low energy shock waves are stimulating the healing, as documented by faster epithelialization, faster wound contraction and faster filling of the excised wound with granulation tissue. As various types of cells are involved in these events, it can be concluded that lithotripsy at a low energy level is nonspecifically activating the function of various cells.

### EXAMPLE 3

Fracture Healing with Acoustic Shock Waves

In order to demonstrate the present invention and the importance of the level of energy input of the shock waves on fracture healing, the following study was conducted on the influence of shock waves on the healing of fractures in Sprague-Dawley rats.

In two experiments a total of 40 Sprague-Dawley rats with a body weight of 200 to 230 g were used. With digital pressure the left humeri were fractured. In each study five treatments were performed 2, 5, 9, 14 and 19 days after infliction of the fracture, using an experimental Dornier XL1 lithotripter. One group of rats were treated with 100 shock waves per application, with either 14 or 18 kilovolts (500 and 800 bar pressure, and 75 and 192 $kW/cm^2$ intensity, respectively). Another group of rats treated in exactly the same fashion except for the application of shock waves served as a control group. The actual focus pressure in the XL1 is higher than in the lithotripters of the HM series, which are used for ESWL and have a different ellipsoid. Table 3 shows the number of rats per treatment group and the experimental design. The various analyzed parameters are shown in Table 4.

#### TABLE 3

Number of Rats per Experiment and Treatment Group

|  | Controls | 14 kV | 18 kV |
|---|---|---|---|
| Experiment 1 | 5 | 7 | 5 |
| Experiment 2 | 11 | 12 | - |

#### TABLE 4

Parameters Analyzed

| Experiment 1 | Experiment 2 |
|---|---|
| Bone Weight | Bone Weight |
| Breaking Strength | Breaking Strength |
|  | Histology |
| Calcium-45-Uptake |  |
| X-ray (at 5 time periods) | X-ray (at 3 time periods) |

In the first experiment X-rays were taken five times, in the second experiment three times, both with industrial high resolution film. The radiographic signs of healing were graded blindly on a subjective scale from 0 to 4 according to a standard scheme by four professionals in an independent fashion. In addition, the callus volume was digitally evaluated. Twenty-four hours before termination, the animals of the first study were injected with 10 microCi calcium-45 per 100 g body weight. After 35 days the rats were sacrificed and both humeri were dissected, carefully cleaned and weighed. The breaking strength of the wet, fresh humeri was measured by longitudinal extension with a tensiometer (Instron table model ®). The calcium-45 uptake was evaluated with a gamma counter. Duncan's multiple range test (supra) was used for the statistical comparisons.

No significant differences were found in the body weight or in the wet bone weights of both humeri between the groups. The fractured bone was uniformly 80 to 110 micrograms heavier than the normal bone, in the treatment groups as well as in the control groups

The non- fractured humeri broke at an average stress of 22.4 kg (standard deviation 3.1). Table 5 shows that the breaking strength of the fractured humeri in the treated groups was higher than in the control groups, absolutely and relative to the bone weight. However, this did not prove to be statistically significant.

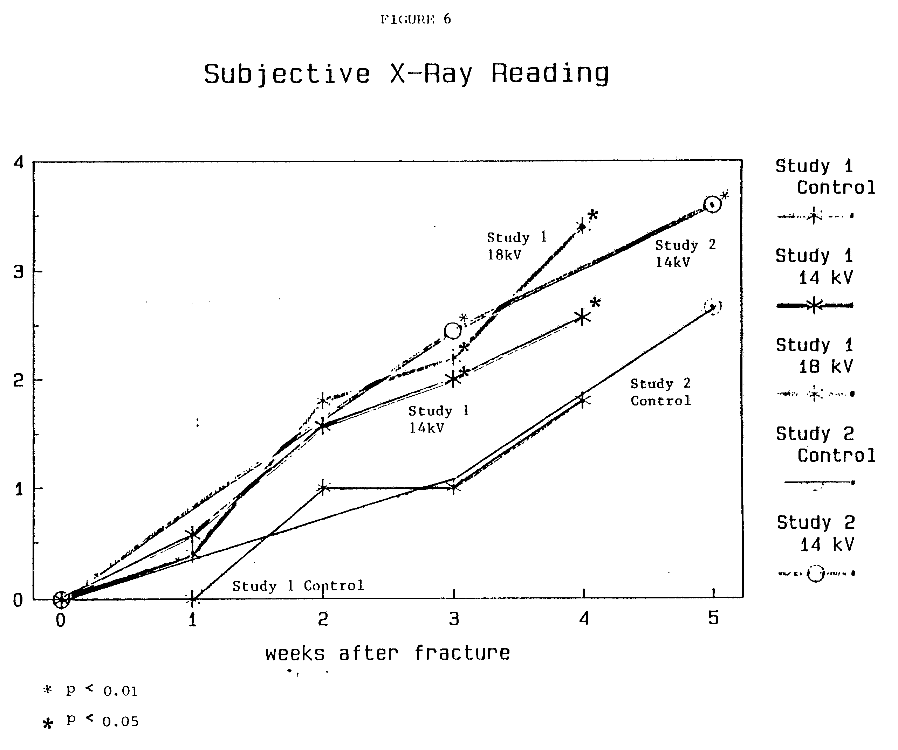

The subjective x-ray readings showed radiological signs of faster healing in the treated groups (Figure 6). These were performed individually and blindly by two trained radiologists and two other physicians with only minor differences among the four readings. The radiographic differences are significant after three or more weeks ($p < 0.05$ in the first and $p < 0.01$ in the second experiment).

In the first experiment the average calcium-45 uptake in the fractured bones was 124% of the uptake in the normal humeri in the untreated group, but only 105% in the 14 kV and 104% in the 18 kV treated groups. (See Table 6).

## TABLE 5

### BREAKING STRENGTH

**BREAKING STRENGTH PER BONE (kg)** $(X \pm SD)$

| | STUDY 1 | | | STUDY 2 | |
|---|---|---|---|---|---|
| | CONTROL | 14kV | 18 kV | CONTROL | 14kV |
| Fractured humerus | 11.6 ± 3.60 | 14.0 ± 3.00 | 14.6 ± 2.48 | 14.1 ± 6.09 | 15.2 ± 4.43 |
| Intact humerus | 22.0 ± 4.98 | 19.6 ± 1.72 | 18.6 ± 1.28 | 24.1 ± 3.55 | 24.9 ± 1.99 |

Breaking Strength in percent of intact humerus

| | | | | |
|---|---|---|---|---|
| 52.7 | 71.4 | 88.2 | 58.5 | 61.0 |

### BREAKING STRENGTH

**BREAKING STRENGTH PER BONE (kg/mg)** $(X \pm SD)$

| | STUDY 1 | | | STUDY 2 | |
|---|---|---|---|---|---|
| | CONTROL | 14kV | 18 kV | CONTROL | 14kV |
| Fractured humerus | 23.8 ± 7.20 | 30.9 ± 7.04 | 30.8 ± 4.68 | 32.3 ± 14.11 | 36.2 ± 9.90 |
| Intact humerus | 54.3 ± 10.34 | 53.2 ± 4.19 | 48.7 ± 3.98 | 43.8 ± 6.85 | 45.9 ± 5.10 |

Breaking Strength in percent of intact humerus

| | | | | |
|---|---|---|---|---|
| 43.8 | 58.1 | 63.2 | 73.7 | 78.9 |

## TABLE 6

### Calcium-45 Uptake

| Study 1 | | | Control | 14 kV | 18 kV |
|---|---|---|---|---|---|
| Calcium-45 Uptake (Counts/g bone) | broken humerus | x | 624 | 386 | 465 |
| | | s | 51 | 204 | 214 |
| | normal humerus | x | 503 | 367 | 449 |
| | | s | 66 | 197 | 198 |

In the second study histological evaluation of the fracture with hematoxylin-eosin, safranin-o and trichrome staining was performed in two samples per treatment group. Although no striking differences could be found between the two groups, some findings should be noted: while in one of the treated humeri the fracture was completely healed, the other treated humerus still revealed more fibrous tissue at the fracture site and partly immature bone. In neither sample hemorrhage or necrosis were found. One of the control humeri showed an inconspicuous, almost completely healed fracture, while the other one represented an immature phase of fracture healing with considerable amounts of cartilage and fibrous tissue. In summary, neither control nor treated samples showed hemorrhage or necrosis, nor significant decreases of hematopoietic cells. Showing only slight differences, the treated samples appeared to heal better.

The results indicate a positive effect of shock waves on fracture healing. Significantly better radiological healing, stronger mechanical stability and some morphological indices in the treated groups suggest enhanced fracture healing. After five weeks the peak of remineralization is passed. Regarding the x-ray results the lower calcium-45 uptake of the treated bones may be interpreted as already normalized remineralization, while the controls are still in a phase of higher mineralization activity. The histological readings prove that neither necrosis, hemorrhage nor bone marrow suppression are visible five weeks after inflicting the fracture or two weeks after the last shock wave treatment; negative effects, which might have been expected after shock wave treatment.

11

It is also interesting to note that the use of relatively low energy shock waves appears to function most efficiently in conjunction with naturally occurring processes. In the above example, the fracture was inflicted by digital pressure, thereby mimicking the manner in whioh fractures typically occur and resulting in the formation of a fracture hematoma. In contrast, experiments with rabbits in which fractures were surgically inflicted by drilling a hole through the tibia or cutting of the fibula (fibula osteotomy) did not show any benefit from the administration of low energy shock waves. Thus, it would appear that the method of the present invention is most advantageously employed to stimulate naturally occurring processes, with higher energy shock waves being necessary when other processes, e.g., surgery, are involved.

While the invention has been described in terms of various preferred embodiments, one skilled in the art will appreciate that various modifications, substitutions, omissions and changes may be made without departing from the spirit thereof. Accordingly, it is intended that the scope of the present invention be limited solely by the scope of the following claims.

## Claims

1. Apparatus for enhancing wound and fracture healing in a mammal comprising a source for acoustic shock waves and a device for coupling them into a site where healing is desired.

2. Apparatus of claim 1 where the amount of energy applied is sufficient to accelerate healing (peak pressure less than about 800 bar preferably < 600 bar, intensity less than about 192 $kW/cm^2$, preferably < 75 $kW/cm^2$).

3. Industrial application (use) of a known non-invasive lithotripter (device for coupling shock waves into a body of a mammal) for enhancing wound or fracture healing.

4. Use of an apparatus for coupling shock waves into a body of a mammal for the manufacture of a device for enhancing wound or fracture healing.

Fig.1

Fig. 2

FIGURE 3

# INFLUENCE OF SHOCKWAVES
# ON THE HEALING OF SPLIT THICKNESS WOUNDS
# IN THE PIG MODEL

Legend: Pig #1, Pig #2, Pig #3

X-axis: Control; 14 kV (10, 100, 500); 18 kV (10, 100)

Y-axis: [%] 0, 20, 40, 60, 80, 100

EP 0 324 711 A2

FIGURE 4

# EFFECT OF SHOCK WAVES ON THE
# CONTRACTION OF DEEP WOUNDS IN PIGLETS

Wound Size
(% from day 0)

Time (Days)

o Control    □ Shock Waves     ◆ Shock Waves
             (46 hrs)          (0, 24 hrs)

EP 0 324 711 A2

FIGURE 5

# EFFECT OF SHOCK WAVES ON GRANULATION TISSUE FORMATION OF DEEP WOUNDS IN PIGLETS

**Wound Volume
(% from day 0)**

Time (Days)

◇ Control    ◻ Shock Waves    ◆ Shock Waves
(46 hrs)        (0, 24 hrs)

EP 0 324 711 A2

FIGURE 6

# Subjective X-Ray Reading

*weeks after fracture*

* P < 0.01

**\*** P < 0.05

EP 0 324 711 A2